# EUROPÄISCHE PATENTSCHRIFT

(11) **EP 3 640 894 B1**
(45) Veröffentlichungstag und Bekanntmachung des Hinweises auf die Patenterteilung: **25.11.2020**
(21) Anmeldenummer: 18201468.8
(22) Anmeldetag: 19.10.2018
(51) Int. Cl.: G06T 11/00

(54) **VERFAHREN ZUR REKONSTRUKTION EINES BILDDATENSATZES DER COMPUTERTOMOGRAPHIE, COMPUTERTOMOGRAPHIEEINRICHTUNG, COMPUTERPROGRAMM UND ELEKTRONISCH LESBARER DATENTRÄGER**
METHOD FOR RECONSTRUCTION OF AN IMAGE DATASET IN COMPUTER TOMOGRAPHY, COMPUTER TOMOGRAPHY DEVICE, COMPUTER PROGRAM AND ELECTRONICALLY READABLE DATA CARRIER
PROCÉDÉ DE RECONSTRUCTION D'UN ENREGISTREMENT DE DONNÉES D'IMAGE D'UNE TOMOGRAPHIE ASSISTÉE PAR ORDINATEUR, DISPOSITIF DE TOMOGRAPHIE ASSISTÉE PAR ORDINATEUR, PROGRAMME INFORMATIQUE ET SUPPORT DE DONNÉES LISIBLE DE MANIÈRE ÉLECTRONIQUE

(43) Veröffentlichungstag der Anmeldung: 22.04.2020
(73) Patentinhaber: Siemens Healthcare GmbH, 91052 Erlangen (DE)
(72) Erfinder: Baer, Matthias, 91054 Erlangen (DE); Hofmann, Christian, 91052 Erlangen (DE); Schöndube, Harald, 91056 Erlangen (DE)

(56) Entgegenhaltungen:
- DE-A1-102013 220 663
- ISMAIL MAHMOUD ET AL: "3D-guided CT reconstruction using time-of-flight camera", MEDICAL IMAGING 2011: VISUALIZATION, IMAGE-GUIDED PROCEDURES, AND MODELING, SPIE, 1000 20TH ST. BELLINGHAM WA 98225-6705 USA, Bd. 7964, Nr. 1, 3. März 2011 (2011-03-03) , Seiten 1-11, XP060008131, DOI: 10.1117/12.878881 [gefunden am 2011-03-01]
- CESARE JENKINS ET AL: "Using a handheld stereo depth camera to overcome limited field-of-view in simulation imaging for radiation therapy treatment planning", MEDICAL PHYSICS., Bd. 44, Nr. 5, 17. April 2017 (2017-04-17) , Seiten 1857-1864, XP055572464, US ISSN: 0094-2405, DOI: 10.1002/mp.12207
- DANIEL KOLDITZ ET AL: "Comparison of extended field-of-view reconstructions in C-arm flat-detector CT using patient size, shape or attenuation information;Comparison of EFOV reconstructions in C-arm FDCT", PHYSICS IN MEDICINE AND BIOLOGY, INSTITUTE OF PHYSICS PUBLISHING, BRISTOL GB, Bd. 56, Nr. 1, 30. November 2010 (2010-11-30), Seiten 39-56, XP020202337, ISSN: 0031-9155, DOI: 10.1088/0031-9155/56/1/003

## Beschreibung

Die Erfindung betrifft ein Verfahren zur Rekonstruktion eines Bilddatensatzes aus unter unterschiedlichen Projektionsrichtungen mit einer Computertomographieeinrichtung aufgenommenen Projektionsbildern eines Untersuchungsobjekts, wobei die Computertomographieeinrichtung ein nominelles Sichtfeld aufweist, das aus allen Projektionsrichtungen in den jeweiligen Projektionsbildern erfasst wird, und wobei das Untersuchungsobjekt und/oder ein dem Untersuchungsobjekt, insbesondere dessen Lagerung, zugeordnetes Zusatzobjekt sich teilweise aus dem nominellen Sichtfeld heraus in ein erweitertes Sichtfeld erstreckt, wobei zur Rekonstruktion des Bilddatensatzes
- eine die Kontur des Untersuchungsobjekts und/oder des Zusatzobjekts beschreibende Konturinformation ermittelt wird,
- die Projektionsdaten der Projektionsbilder aufgrund der Konturinformation durch Vorwärtsprojektion in Bereichen, in denen das Untersuchungsobjekt und/oder das Zusatzobjekt in den Projektionsdaten eines Projektionsbildes nicht erfasst wurde, ergänzt werden, und
- die Rekonstruktion aufgrund der ergänzten Projektionsdaten erfolgt. Daneben betrifft die Erfindung eine Computertomographieeinrichtung, ein Computerprogramm und einen elektronisch lesbaren Datenträger.

In der Computertomographie werden niederdimensionale Projektionsbilder, beispielsweise ein- oder zweidimensionale Projektionsbilder, eines Untersuchungsobjekts aus unterschiedlichen Projektionsrichtungen, beispielsweise entlang einer Aufnahmetrajektorie, insbesondere einer Kreisbahn, aufgenommen. Mittels bekannter Rekonstruktionsverfahren, beispielsweise der gefilterten Rückprojektion, können aus den eindimensionalen oder zweidimensionalen Projektionsbildern zweidimensionale Schnittbilder bzw. dreidimensionale Volumina als Bilddatensätze rekonstruiert werden. Für eine artefaktfreie Rekonstruktion ist es dabei wichtig, dass das Untersuchungsobjekt senkrecht zum Verlauf der Aufnahmetrajektorie in seiner vollen Ausdehnung durch alle Projektionsbilder erfasst wird, wobei in üblichen Computertomographieeinrichtungen der Raumbereich, der in allen Projektionsrichtungen erfasst wird, auch als nominelles Sichtfeld bzw. englisch als "scan Field of View" (sFoV) bezeichnet wird. Das nominelle Sichtfeld bezeichnet mithin den Bereich innerhalb der Computertomographie-Gantry, der durch den Röntgendetektor der Aufnahmeanordnung der Computertomographieeinrichtung vollständig abgedeckt wird.

Es existieren jedoch Untersuchungssituationen, in denen sich Teile des Patienten als Untersuchungsobjekt außerhalb des nominellen Sichtfelds befinden und somit mit herkömmlichen Rekonstruktionsalgorithmen nicht bzw. nicht artefaktfrei rekonstruiert werden können. Dies tritt beispielsweise dann ein, wenn die Computertomographie-Bildgebung für die Erstellung von Bestrahlungsplänen für die Strahlentherapie eingesetzt wird. Dabei werden häufig als Zusatzobjekte spezielle Lagerungshilfen eingesetzt, beispielsweise Kopffixiermittel oder Brusthalterungen. Durch die Verwendung der Lagerungshilfen sind die Möglichkeiten zur Positionierung des Patienten innerhalb der Computertomographieeinrichtung, insbesondere der Gantry, deutlich eingeschränkt, so dass nicht immer verhindert werden kann, dass sich relevante Teile des Patienten außerhalb des nominellen Sichtfelds befinden. Neben der Verwendung von speziellen Zusatzobjekten, insbesondere Lagerungshilfen, kann auch der Patientenhabitus, beispielsweise bei fettleibigen Patienten, dazu führen, dass sich relevante Bereiche des Patienten außerhalb des nominellen Sichtfelds befinden. Das nominelle Sichtfeld kann beispielsweise einen Durchmesser von 500 mm aufweisen.

Um auch nicht in allen Projektionsbildern erfasste Anteile des Patienten im Bilddatensatz direkt und genau darstellen zu können und somit eine korrekte Diagnose bzw. eine korrekte Berechnung von Bestrahlungsplänen auch in Bereichen außerhalb des nominellen Sichtfelds zu gewährleisten, wurden im Stand der Technik bereits verschiedene Verfahren zu einer sogenannten Rekonstruktion im erweiterten Sichtfeld (Extended Field-of-View-Rekonstruktion) vorgeschlagen. Eine dabei bekannte Klasse von Vorgehensweisen sieht vor, dass zunächst eine initiale Schätzung der Kontur des Untersuchungsobjekts, meist des Patienten, auch in Bereichen außerhalb des nominellen Sichtfelds als Konturinformation ermittelt wird. Diese Konturinformation wird dann in einem weiteren Schritt dazu verwendet, Projektionsdaten der Projektionsbilder außerhalb des nominellen Sichtfelds zu ergänzen, um dann eine möglichst genaue Rekonstruktion des Bilddatensatzes auch in einem erweiterten Sichtfeld, das größer als das nominelle Sichtfeld ist, zu erlauben.

Dabei ist es beispielsweise bekannt, zunächst eine initiale Rekonstruktion eines Vorabdatensatzes aus den Projektionsdaten vorzunehmen, wobei Extrapolationsverfahren verwendet werden können, die ein Auftreten von sogenannten Trunkierungsartefakten verhindern, so dass die gemessenen Computertomographiedaten in Bereiche außerhalb des Röntgendetektors extrapoliert werden können, die bei der Messung nicht erfasst wurden. Hierzu sei beispielsweise auf den Artikel von J. Hsieh et al., "A novel reconstruction algorithm to extend the CT scan field-of-view", Med. Phys. 31(9), Seiten 2385 - 2391 (2004), und den Artikel von B. Ohnesorge et al., "Efficient correction for CT image artifacts caused by objects extending outside the scan field of view", Med. Phys. 27(1), Seiten 39 - 46 (2000), verwiesen.

Nach einer initialen Rekonstruktion eines Vorabdatensatzes kann die Kontur des Untersuchungsobjekts, konkret des Patienten, beispielsweise anhand von Schwellwertverfahren erfolgen, so dass mithin die Konturinformation ein binäres Bild des Patienten sein kann, welche den initial rekonstruierten Bilddatensatz typischerweise in Bereiche klassifiziert, die Luft enthalten, und solche, die das Untersuchungsobjekt und/oder gegebenenfalls Zusatzobjekte, beispielsweise Lagerungshilfen, enthalten. Derartige Konturinformationen, insbesondere binäre Bilder, können genutzt werden, um durch Vorwärtsprojektion und fehlende Projektionsdaten in den Projektionsbildern möglichst originalgetreu zu ergänzen, beispielsweise, indem im Rahmen der Vorwärtsprojektion diesen Patientenvoxeln für Patienten übliche HU-Werte, beispielsweise die von Wasser, zugeordnet werden. Mit aufgrund einer derartigen Vorwärtsprojektion ergänzten Projektionsdaten bzw. Projektionsbildern ist auch in Bereichen außerhalb des nominellen Sichtfelds ein möglichst korrekter Bilddatensatz erhaltbar. Zur Ermittlung einer Konturinformation aus einem in einer initialen Rekonstruktion erhaltenen Rekonstruktionsdatensatz sei insbesondere auf US 2011/0 188 723 A1 und US 9,495,769 B2 verwiesen.

Nichtsdestotrotz sind derartige initiale Rekonstruktionen, die aufgrund der ursprünglich aufgenommenen Projektionsbilder erfolgen, nicht gänzlich artefaktfrei bzw. können Fehler insbesondere in Bereichen erhalten, die nur in sehr wenigen Projektionsbildern (oder überhaupt nicht) in den ursprünglichen Projektionsdaten enthalten sind.

In einem Artikel von Mahmoud Ismail et al., "3D-Guided CT-Reconstruction using Time-Of-Flight Cameras", Medical Imaging 2011: Visualization, Image-Guided Procedures, and Modeling, SPIE, Band 7964, Nr. 1, 3. März 2011, Seiten 1-11, wird die Benutzung einer Time-of-Flight-Kamera (TOF-Kamera) vorgeschlagen, um die Körperkontur eines Patienten für ein dreidimensionales geführtes Rekonstruktionsschema für CT- und C-Bogensysteme mit trunkierter Projektion zu ermitteln. Projektionsdaten in nicht vermessenen Bereichen werden abgeschätzt und ergänzt.

Der Erfindung liegt daher die Aufgabe zugrunde, die Rekonstruktionsqualität bei Rekonstruktionen von Computertomographie-Bilddatensätzen in erweiterten Sichtfeldern, die größer als das nominelle Sichtfeld sind, zu verbessern.

Diese Aufgabe wird gelöst durch ein Verfahren, eine Computertomographieeinrichtung, ein Computerprogramm und einen elektronisch lesbaren Datenträger gemäß den unabhängigen Ansprüchen. Vorteilhafte Weiterbildungen ergeben sich aus den Unteransprüchen.

In einem Verfahren der eingangs genannten Art ist erfindungsgemäß vorgesehen, dass aus Sensordaten wenigstens einer das zur Aufnahme der Projektionsbilder positionierte Untersuchungsobjekt und/oder das Zusatzobjekt erfassenden Kamera eine die Kontur des Untersuchungsobjekts und/oder des Zusatzobjekts wenigstens teilweise beschreibende Sensorinformation ermittelt und zur Ermittlung der Konturinformation verwendet wird.

Nachdem die Computertomographie hauptsächlich im Bereich der Medizin angewendet wird, handelt es sich bei dem Untersuchungsobjekt insbesondere um einen Patienten. Daher wird sich im Folgenden eine Vielzahl von Beispielen auf einen Patienten als Untersuchungsobjekts beziehen, wobei die vorliegende Erfindung jedoch auch auf andere Untersuchungsobjekte, beispielsweise in der Materialprüfung, anwendbar ist.

Erfindungsgemäß wird also vorgeschlagen, die Kontur des Untersuchungsobjekts bzw., falls ein Zusatzobjekt aus dem nominellen Sichtfeld herausragt, des Zusatzobjekts nicht oder zumindest nicht ausschließlich aufgrund einer initialen Rekonstruktion aus den ursprünglich aufgenommenen Projektionsbildern zu ermitteln, sondern auf eine zusätzliche Sensorik, vorliegend insbesondere optische Kameras, die in der Nähe der Computertomographieeinrichtung bzw. der Untersuchungsobjektlagerungsstätte angeordnet sind, einzusetzen, um so genauere und verlässlichere Informationen über die entsprechenden Konturen in Form der Sensorinformation zu erhalten und zur Ermittlung der Konturinformation zu nutzen. Das bedeutet im konkreten Fall, dass sich die vorliegende Erfindung auf die Ermittlung der Konturinformation, die in den im Stand der Technik grundsätzlich bekannten Verfahren zur Rekonstruktion im erweiterten Sichtfeld benötigt wird, bezieht. Konkret wird in einem Beispiel vorgeschlagen, die Patientenkontur unter Verwendung einer oder mehrerer zusätzlicher Kameras, die beispielsweise an der Decke oberhalb eines Patiententisches angeordnet sein können, zu bestimmen. Mithilfe von Kameras ist es möglich, die reale Oberfläche des Untersuchungsobjekts und deren Position im Raum während des Untersuchungsvorgangs mit der Computertomographieeinrichtung zu erfassen. Diese zusätzlichen Sensordaten, aus denen als Sensorinformation die Lage und Form der Kontur ermittelt werden kann, werden dann in Form der Sensorinformation genutzt, um die Konturinformation zu ermitteln, welche dann genutzt wird, um Projektionsbilder möglichst korrekt zu ergänzen und im Rahmen der Rekonstruktion des Bilddatensatzes hochqualitative, möglichst artefaktfreie Informationen zu erhalten. Die Vermessung durch Kameras erlaubt es, die gemessenen Projektionsdaten in Bereichen, die nicht vom Röntgendetektor erfasst wurden, möglichst korrekt, das heißt in möglichst hoher Übereinstimmung mit der Realität, zu ergänzen.

Eine verbesserte Bildqualität von Rekonstruktionen in einem gegenüber dem nominellen Sichtfeld erweiterten Sichtfeld (extended field of view - eFoV) kann dazu beitragen, dass auch Computertomographieeinrichtungen, deren nominelles Sichtfeld gegenüber anderen Computertomographieeinrichtungen eingeschränkt ist, konkurrenzfähig werden, nachdem auch über das nominelle Sichtfeld hinaus Bilddatensätze hervorragender Qualität rekonstruiert werden können.

Zweckmäßigerweise kann eine als eine 3D-Kamera, insbesondere eine optische oder Terahertz-Kamera, ausgebildete Kamera verwendet werden und/oder wenigstens eine auf die Kontur des Untersuchungsobjekts und/oder des Zusatzobjekts bezogene 3D-Information aus Sensordaten wenigstens zweier unterschiedlich positionierter Kameras und/oder aus einer Anordnungsinformation der Kamera ermittelt werden. Bevorzugt werden im Rahmen der vorliegenden Erfindung 3D-Kameras, beispielsweise also optische 3D-Kameras und/oder Terahertz-Kameras, eingesetzt.

Diese liefern unmittelbar dreidimensionale Sensordaten bezüglich der durch sie erfassten Objekte, insbesondere also des Untersuchungsobjekts und/oder des Zusatzobjekts. Denkbar ist es jedoch auch, beispielsweise dreidimensionale Informationen aufgrund von das Untersuchungsobjekt und/oder das Zusatzobjekt aus unterschiedlichen Richtungen erfassenden zweidimensionalen Kameras, beispielsweise stereoskopischen Kameras, zu ermitteln oder gar unter Kenntnis der Anordnung und Erfassungsgeometrie bereits aus den Daten einer einzigen zweidimensionalen Kamera auf relevante dreidimensionale Informationen schließen. Wie bereits erwähnt, ist es im Rahmen der Erfindung jedoch bevorzugt, 3D-Kameras, insbesondere optische 3D-Kameras, zu verwenden. Die Verwendung optischer Kameras hat den weiteren Vorteil, dass die Sensordaten auch hinsichtlich optischer Informationen ausgewertet werden kann, beispielsweise zur Identifikation spezieller Zusatzobjekte, was im Folgenden noch näher diskutiert werden wird.

In zweckmäßiger Weiterbildung kann zudem vorgesehen sein, dass außerhalb des Erfassungsbereiches einer Kamera liegende Anteile der Kontur durch wenigstens eine weitere Kamera erfasst werden. Beispielsweise kann es durch die Lagerung bzw. die Anatomie eines Patienten als Untersuchungsobjekt dazu kommen, dass Anteile des Patienten bzw. von dessen Oberfläche nicht von einer einzigen Kamera erfasst werden können. Ähnliches kann für Zusatzobjekte gelten. Um dennoch eine hinreichende Informationsmenge zu gewährleisten, können mehrere, an unterschiedlichen Stellen im Raum angeordnete, entsprechende Erfassungsbereiche aufweisende Kameras verwendet werden.

Zweckmäßigerweise kann vorgesehen sein, dass die Kamera mit einer Aufnahmeanordnung der Computertomographieeinrichtung registriert ist und/oder wird und/oder bei mehreren Kameras diese untereinander registriert sind und/oder werden. Bei einer Registrierung können in der Sensorinformation enthaltene Ortsinformationen unmittelbar auch im Koordinatensystem angewendet werden, in dem die Computertomographieaufnahme stattfindet. Zumindest ergeben sich bei einer Registrierung oder dergleichen hervorragende Ausgangspositionen. Dabei ist sowohl eine permanent vorhandene Registrierung denkbar, die auf einer festen Anordnung der Kamera zu sonstigen Komponenten der Computertomographieeinrichtung beruht. Auf der anderen Seite ist es jedoch selbstverständlich auch möglich, eine Registrierung explizit vorzunehmen, beispielsweise durch Verwendung von sowohl in den Sensordaten der Kamera als auch in Röntgenbildern der Computertomographieeinrichtung sichtbaren Phantomen und/oder Markern. Es sei an dieser Stelle noch angemerkt, dass nicht zwangsläufig eine Registrierung zwischen der Kamera und der Aufnahmeanordnung der Computertomographieeinrichtung bestehen muss, nachdem beispielsweise Oberflächen von Untersuchungsobjekten zur Durchführung von Registrierungen auch aufgrund einer groben bekannten Positionsbeziehung leicht mit beispielsweise im Rahmen einer initialen Rekonstruktion aus den ursprünglichen Projektionsdaten gewonnenen Vorabinformation über die Konturen registriert werden können.

Vorzugsweise kann ein nicht durch die wenigstens eine Kamera erfassbarer, insbesondere verdeckter Anteil der Kontur des Untersuchungsobjekts durch Extrapolation und/oder Interpolation, insbesondere unter Verwendung eines Patientenmodells, ermittelt werden. Das bedeutet, beispielsweise bei Anteilen der Oberfläche eines Patienten, die durch den Patiententisch und/oder Zusatzobjekte und/oder andere Anteile des Patienten verdeckt sind, können fehlende Informationen beispielsweise durch geeignete Inter- bzw. Extrapolationsmethoden ermittelt werden. Besonders bevorzugt ist es jedoch, ein insbesondere statistisches Patientenmodell zu verwenden, welches die äußere Kontur eines durchschnittlichen Patienten beschreibt, welches dann in den bekannten Abschnitten der Kontur des Untersuchungsobjekts auf den aktuellen Patienten angepasst werden kann, so dass auf die fehlende Anteile der Kontur rückgeschlossen werden kann. Es sei angemerkt, dass derartige unbekannte Anteile auch von der weiteren Verwendung ausgeschlossen werden können, beispielsweise also als unbekannt markiert werden können.

In konkreter Ausgestaltung der vorliegenden Erfindung kann vorgesehen sein, dass als Konturinformation eine wenigstens nach Luft und Untersuchungsobjekt und/oder Zusatzobjekt klassifizierende binäre Einteilung wenigstens des erweiterten Sichtfelds verwendet wird. Das bedeutet, die Konturinformation kann als eine Art "binäres Bild" oder im Allgemeinen "Materialklassen-Bild" vorliegen, nachdem sich Derartiges hervorragend als Ausgangspunkt für eine Vorwärtsprojektion eignet, sei es, in dem als "Objekt" markierte Bereiche als entsprechenden HU-Werten befüllt werden, oder sei es, dass es lediglich um die Bestimmung der Begrenzung des Untersuchungsobjekts und/oder des Zusatzobjekts innerhalb der zu ergänzenden Projektionsbilder bestimmter Projektionsrichtungen geht. Letztlich kann ein derartiges binäres Bild als in zwei Materialklassen eingeteilt verstanden werden, Es sei darauf hingewiesen, dass auch Ausgestaltungen denkbar und vorteilhaft sind, in denen mehr als zwei Materialklassen verwendet werden. Insbesondere kann beispielsweise auch zwischen Zusatzobjekten und Untersuchungsobjekt unterschieden werden, was insbesondere dann zweckmäßig ist, wenn das Zusatzobjekt eine von dem Patienten deutlich unterschiedliche Schwächungseigenschaft aufweist, die jedoch bekannt ist. Auch können ggf. unterschiedliche Materialarten im Zusatzobjekt über mehrere Materialklassen abgebildet werden.

Grundsätzlich ist es im Rahmen der vorliegenden Erfindung vorteilhaft denkbar, dass die Ermittlung der Konturinformation ausgehend von der Sensorinformation oder sogar, wenn die über das nominale Sichtfeld herausragenden Anteile des Untersuchungsobjekts und/oder des Zusatzobjekts vollständig und insbesondere hinreichend genau durch die Sensordaten beschrieben werden, als die Sensorinformation erfolgt. Nachdem davon ausgegangen werden kann, dass die Sensordaten das Untersuchungsobjekt und/oder das Zusatzobjekt hinreichend genau und verlässlich wiedergeben, kann insbesondere bei vollständiger Wiedergabe des wenigstens einen Objekts mithin bereits unmittelbar als Sensorinformation beispielsweise ein binäres Bild bzw. ein in mehr als zwei Materialklassen klassifiziertes Bild ermittelt werden und der Vorwärtsprojektion zugrunde gelegt werden. Denkbar ist es jedoch auch, beispielsweise eine Ergänzung der Sensorinformation aus einer aus einer vorläufigen Rekonstruktion aus den ursprünglichen Projektionsbildern ermittelten Vorabinformation zur Ermittlung der Konturinformation vorzunehmen.

Die Erfindung sieht vor, dass zunächst durch Rekonstruktion eines vorläufigen Rekonstruktionsdatensatzes aus den ursprünglichen Projektionsbildern für das erweiterte Sichtfeld und Schwellwertbildung eine wenigstens die Kontur des Untersuchungsobjekts und/oder des Zusatzobjekts vorläufig beschreibende Vorabinformation ermittelt wird, welche aufgrund der Sensorinformation angepasst wird. Im Beispiel eines Patienten kann ein binäres Bild bzw. allgemein ein in mehrere Materialklassen klassifizierendes Bild (Materialklassen-Bild), insbesondere eine Klassifikation von Voxeln in Luft und Patient, auch aus einer initialen Rekonstruktion der gemessenen, ursprünglichen Projektionsbilder, insbesondere mittels Schwellwertbildung, erstellt werden. Diese initiale Rekonstruktion kann beispielsweise wie im eingangs genannten Stand der Technik speziell für erweiterte Sichtfelder durchgeführt werden, um beispielsweise Trunkierungsartefakte zu verringern bzw. zu vermeiden. Allerdings kann auch eine einfache initiale Rekonstruktion nur basierend auf den gemessenen Projektionsdaten erfolgen.

Die aus den Sensordaten der wenigstens einen Kamera gewonnene Sensorinformation über Lage und Form der Oberfläche des Untersuchungsobjekts, im Beispiel des Patienten, kann nun dazu verwendet werden, die aus der Schwellwertbildung ermittelte Kontur des Untersuchungsobjekts, vor allem in Bereichen außerhalb des nominellen Sichtfelds der Computertomographieeinrichtung, zu verbessern. Hierzu kann insbesondere vorgesehen sein, dass die Anpassung der Vorabinformation zu der Konturinformation durch elastische Registrierung der Vorabinformation auf in der Sensorinformation enthaltene Anteile der Kontur erfolgt. Es kann also beispielsweise die aus der initialen Rekonstruktion der ursprünglichen Projektionsdaten erstellte Kontur des Untersuchungsobjekts mittels eines geeigneten Registrierungsalgorithmus so angepasst werden, dass die Kontur des Untersuchungsobjekts nach der Registrierung mit der Lage der aus den Sensordaten der wenigstens einen Kamera bestimmten Untersuchungsobjektoberfläche, insbesondere Patientenoberfläche, übereinstimmt. Entsprechendes kann selbstverständlich auch bezüglich von Zusatzobjekten durchgeführt werden, deren Oberfläche ebenso aus den Sensordaten der wenigstens einen Kamera abgeschätzt und als Sensorinformation genutzt werden kann. Falls Teile einer Oberfläche bzw. Kontur nicht durch die wenigstens eine Kamera erfasst werden können und keine geeignete Interpolation möglich erscheint, können diese Teile auch von der Registrierung ausgeschlossen werden, wobei jedoch eine elastische Registrierung dennoch zumindest für einen glatten Übergang sorgen kann.

Bezüglich von Zusatzobjekten sei an dieser Stelle noch angemerkt, dass Artefakte in Bilddatensätzen auch bereits dann auftreten können, wenn zwar das Untersuchungsobjekt vollständig im nominellen Sichtfeld enthalten ist, aber ein Zusatzobjekt, beispielsweise zur Lagerung des Untersuchungsobjekts, wie beispielsweise ein Kopffixiermittel für einen Patienten, aus dem nominellen Sichtfeld herausragt. Insofern muss sich nicht zwangsläufig die Konturbestimmung (ausschließlich) auf das Untersuchungsobjekt, insbesondere den Patienten, beziehen, sondern kann auch Zusatzobjekte, beispielsweise Kopffixiermittel, Brusthalterungen und dergleichen, betreffen.

Neben der direkten Verwendung von Sensordaten der wenigstens einen Kamera bezüglich der Untersuchungsobjektoberfläche können die Sensordaten der wenigstens einen Kamera also auch dazu verwendet werden, bestimmte Zusatzobjekte, die sich im erweiterten Sichtfeld befinden, zu erkennen. Dies ist, wie im Folgenden dargelegt wird, besonders bei Zusatzobjekten zweckmäßig, für die exakte dreidimensionale Computermodelle, beispielsweise CAD-Modelle und/oder Konstruktionszeichnungen, existieren. Dies ist beispielsweise bei Lagerungshilfen und/oder Patiententischen als Zusatzobjekte häufig der Fall. Dann kann auch ein derartiges dreidimensionales Computermodell des Zusatzobjekts zweckmäßig genutzt werden, um die Berechnung der Konturinformation zu verbessern.

Eine Weiterbildung der vorliegenden Erfindung sieht entsprechend vor, dass aus den Sensordaten wenigstens eine die Art des insbesondere zur Lagerung und/oder zur Positionierung des Untersuchungsobjekts verwendeten Zusatzobjekts, zu dem ein dreidimensionales Computermodell, insbesondere ein CAD-Modell, vorliegt, beschreibende Objektinformation ermittelt wird, wobei das dreidimensionale Computermodell bei der Ermittlung der die Kontur des Zusatzobjekts beschreibenden Konturinformation verwendet wird. Dabei kann konkret vorgesehen sein, dass aufgrund der Objektinformation das dreidimensionale Computermodell des Zusatzobjekts ausgewählt wird, wonach die Kontur des Zusatzobjekts gemäß der Sensorinformation und/oder der Vorabinformation zur Ermittlung der Kontur des Zusatzobjekts in der Konturinformation an das dreidimensionale Computermodell des Zusatzobjekts, insbesondere in seiner Lage und/oder durch starre oder elastische Registrierung, angepasst wird. Insbesondere kann hierbei anhand der Sensorinformation und/oder der Vorabinformation also die am besten übereinstimmende Lage des als starr angenommenen dreidimensionalen Computermodells bestimmt werden, wonach die durch das dreidimensionale Computermodell in dieser Lage beschriebene Oberfläche als Kontur des Zusatzobjekts in der Konturinformation verwendet wird. Dabei sei an dieser Stelle darauf hingewiesen, dass die Objektinformation besonders einfach ermittelt werden kann, wenn mittels der wenigstens einen Kamera optische Sensordaten aufgenommen werden, da dies wichtige Informationen zur Identifikation eines Zusatzobjekts liefert.

Wurde also ein Zusatzobjekt mithilfe der wenigstens einen Kamera erkannt, kann dessen dreidimensionales Computermodell, beispielsweise CAD-Modell, entsprechend zur Verbesserung der Konturinformation herangezogen werden, insbesondere indem das dreidimensionale Computermodell an eine initiale Rekonstruktion der ursprünglichen Projektionsbilder, wie oben als Vorabinformation beschrieben, bezüglich seiner Lage im Raum angepasst wird. Aus dem bezüglich seiner Lage an die initiale Rekonstruktion angepassten dreidimensionalen Computermodell kann dann wiederum ein binäres Bild bzw. ein Bild mit mehreren Materialklassen erstellt werden, welches, falls nötig, auch eine Kontur des Untersuchungsobjekts enthalten kann, dann aber bevorzugt Voxel auch entweder dem Zusatzobjekt und/oder dem Untersuchungsobjekt zuordnet.

Dabei sei an dieser Stelle angemerkt, dass eine Nutzung des dreidimensionalen Computermodells des Zusatzobjekts auch bei einer unmittelbaren Ermittlung der Konturinformation aus der Sensorinformation zweckmäßig ist, da das dreidimensionale Computermodell als die Realität wiedergebend verstanden werden kann und somit auch beispielsweise Messfehler oder dergleichen in den Sensordaten der wenigstens einen Kamera ausgleichen kann, indem es in die in der Sensorinformation bestimmte Kontur des Zusatzobjekts eingepasst wird und diese ersetzt. Dabei ist es besonders zweckmäßig, dass aufgrund des dreidimensionalen Modells sowohl bei dessen Nutzung in der Sensorinformation als auch bei dessen Nutzung in der Vorabinformation auch durch die Kamera nicht einsehbare Begrenzungen des Zusatzobjekts bekannt werden, was insbesondere dann zweckmäßig ist, wenn bei der Vorwärtsprojektion mit zugeordneten HU-Werten gearbeitet werden soll, wobei dem Zusatzobjekt und dem Untersuchungsobjekt unterschiedliche HU-Werte zuzuordnen sind.

In diesem Kontext ist es auch besonders vorteilhaft, wenn das dreidimensionale Computermodell des Zusatzobjekts auch eine Schwächungseigenschaften für Röntgenstrahlung in dem Zusatzobjekt beschreibende Materialinformation umfasst, welche bei der Vorwärtsprojektion berücksichtigt wird. Falls also neben dem geometrischen Modell des Zusatzobjektes auch Information über dessen Materialzusammensetzung vorliegt, kann diese dazu verwendet werden, das Konturbild des Zusatzobjekts für die Vorwärtsprojektion weiter zu verbessern, indem Bereiche innerhalb des Zusatzobjekts auf den korrekten HU-Wert gesetzt werden können. Insbesondere bei der Bestrahlungsplanung ist die Korrektheit der HU-Werte (bzw. allgemein Schwächungswerte) von großer Bedeutung, da die Korrektheit der Bestrahlungsplanung, insbesondere was die Dosisverteilung betrifft, in direktem Zusammenhang zur Korrektheit der HU-Werte ("Hounsfield Units") steht.

Wie bereits erläutert, können basierend auf der mithilfe der Sensordaten der wenigstens einen Kamera ermittelten Konturinformation mittels Vorwärtsprojektion virtuelle Projektionsdaten erstellt werden, welche in einem weiteren Schritt dazu verwendet werden können, die gemessenen ursprünglichen Projektionsdaten in Bereichen außerhalb des nominellen Sichtfelds zu ergänzen.

In einer besonders bevorzugten Weiterbildung der vorliegenden Erfindung kann vorgesehen sein, dass für jede Messung mit der Computertomographieeinrichtung die Sensorinformation automatisch auf aus dem nominellen Sichtfeld ragende Anteile des Untersuchungsobjekts und/oder des Zusatzobjekts überprüft wird, wodurch automatisch die Rekonstruktion mit oder ohne erweitertes Sichtfeld ausgelöst wird. Das bedeutet, die Auswertung der Sensordaten der wenigstens einen Kamera kann für jede Untersuchung mit der Computertomographieeinrichtung erfolgen, um diese Untersuchungen in solche, die ein erweitertes Sichtfeld benötigen, und solche, für die das nominelle Sichtfeld ausreichend ist, zu klassifizieren. Das bedeutet, die Sensorinformation über Lage und Form des Untersuchungsobjekts bzw. von Zusatzobjekten kann dazu verwendet werden, einen Algorithmus zur Rekonstruktion in einem erweiterten Sichtfeld automatisiert an- bzw. auszuschalten. Aktuell wird dieser Schritt typischerweise manuell durch einen Benutzer der Computertomographieeinrichtung durchgeführt. Mithin wird die Entscheidung über die Notwendigkeit des Einsatzes eines speziellen Algorithmus zur Rekonstruktion im erweiterten Sichtfeld automatisiert anhand der Sensordaten der wenigstens einen Kamera getroffen.

Neben dem Verfahren betrifft die vorliegende Erfindung auch eine Computertomographieeinrichtung, aufweisend wenigstens eine Kamera und eine zur Durchführung des erfindungsgemäßen Verfahrens ausgebildete Steuereinrichtung. Sämtliche Ausführungen bezüglich des erfindungsgemäßen Verfahrens lassen sich analog auf die erfindungsgemäße Computertomographieeinrichtung übertragen, mit welcher mithin ebenso die bereits genannten Vorteile erhalten werden können. Insbesondere kann die Computertomographieeinrichtung eine Aufnahmeanordnung, umfassend einen Röntgenstrahler und einen Röntgendetektor, aufweisen, wobei die Aufnahmeanordnung beispielsweise bewegbar in einer Gantry gelagert sein kann. Zur Lagerung des Untersuchungsobjekts kann die Computertomographieeinrichtung ferner einen Patiententisch aufweisen. Die Steuereinrichtung kann wenigstens einen Prozessor und wenigstens ein Speichermittel aufweisen. Konkret kann die Steuereinrichtung zur Durchführung des erfindungsgemäßen Verfahrens beispielsweise eine Rekonstruktionseinheit zur Rekonstruktion von Bilddatensätzen und/oder Rekonstruktionsdatensätzen für Vorabinformationen, eine Sensorinformationsermittlungseinheit, eine Konturinformationsermittlungseinheit, eine Vorwärtsprojektionseinheit und eine Ergänzungseinheit aufweisen.

Ein erfindungsgemäßes Computerprogramm ist beispielsweise direkt in einen Speicher einer Steuereinrichtung einer Computertomographieeinrichtung ladbar und weist Programmmittel auf, um die Schritte eines erfindungsgemäßen Verfahrens auszuführen, wenn das Computerprogramm in der Steuereinrichtung der Computertomographieeinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein erfindungsgemäßes Computerprogramm umfassen und derart ausgestaltet sind, dass sie bei Verwendung des Datenträgers in einer Steuereinrichtung einer Computertomographieeinrichtung ein erfindungsgemäßes Verfahren durchführen. Bei dem Datenträger kann es sich insbesondere um einen nichttransienten Datenträger, beispielsweise eine CD-ROM, handeln.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen Ablaufplan eines Ausführungsbeispiels des erfindungsgemäßen Verfahrens,
- Fig. 2: eine Skizze zur Erläuterung der Trunkierung eines Untersuchungsobjekts,
- Fig. 3: die elastische Registrierung der Kontur einer Vorabinformation auf eine Kontur einer Sensorinformation,
- Fig. 4: eine schematische Erläuterung der Verwendung eines dreidimensionalen Computermodells eines Zusatzobjekts,
- Fig. 5: eine erfindungsgemäße Computertomographieeinrichtung und
- Fig. 6: den funktionalen Aufbau der Steuereinrichtung der Computertomographieeinrichtung der Fig. 5.

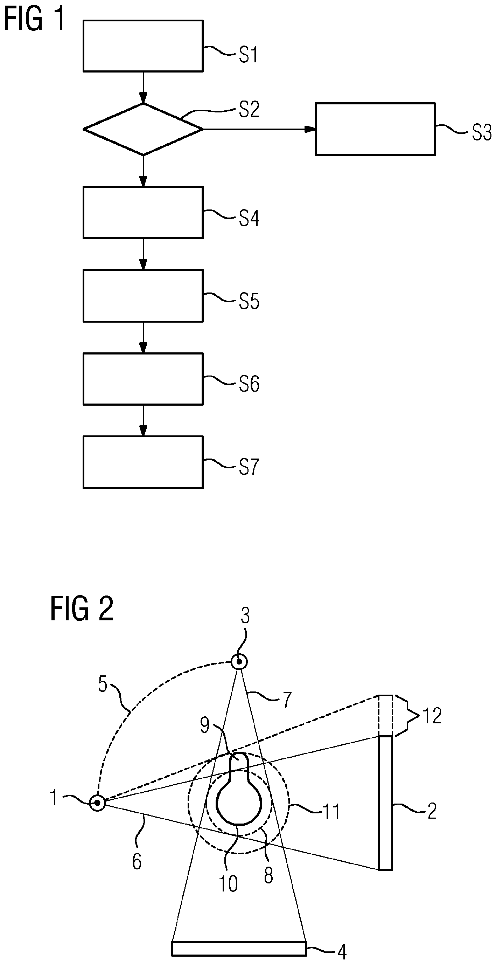

Fig. 1 zeigt einen Ablaufplan eines Ausführungsbeispiels des erfindungsgemäßen Verfahrens. Dieses wird an einer Computertomographieeinrichtung durchgeführt, welche beispielsweise eine in einer Gantry bewegte Aufnahmeanordnung mit einem Röntgendetektor und einem Röntgenstrahler aufweist. Ein Patient als Untersuchungsobjekt kann auf einer Patientenlagerungseinrichtung, insbesondere einem Patiententisch, gelagert werden. Hierbei können als Zusatzobjekte Lagerungshilfen, beispielsweise Kopffixierungen und/oder Brusthalterungen, eingesetzt werden. Die Computertomographieeinrichtung weist ein, beispielsweise zylinderförmiges, nominelles Sichtfeld auf, in dem Projektionsdaten aus allen Projektionsrichtungen der Aufnahmeanordnung aufgenommen werden können.

Der Computertomographieeinrichtung sind ferner ein oder mehrere optische 3D-Kameras zugeordnet, die Sensordaten eines auf der Patientenlagerungseinrichtung angeordneten Patienten, insbesondere im Bereich des nominellen Sichtfelds, aufnehmen können.

In einem zu jeder Untersuchung eines Patienten durchgeführten Schritt S1 wird eine Sensorinformation bestimmt, die den Verlauf von Oberflächen, also Konturen, des Patienten sowie gegebenenfalls eines zumindest teilweise im nominellen Sichtfeld befindlichen Zusatzobjekts, beispielsweise einer Lagerungshilfe, beschreiben. Hierzu werden die dreidimensional vorliegenden Sensordaten entsprechend ausgewertet.

Dabei sind verschiedene Repräsentationen der Sensorinformation denkbar, beispielsweise eine Beschreibung der Oberflächen, die die Konturen bilden, an sich, bevorzugt ist es jedoch, ein binäres oder sogar ein in mehr als zwei Materialklassen eingeteiltes Bild als Sensorinformation zu verwenden. Ein binäres Bild wird dabei zwischen "Luft" und "Material" (also Untersuchungsobjekt bzw. Zusatzobjekt) unterscheiden; ein mehr Materialklassen nutzendes Bild hat den Vorteil, dass beispielsweise auch zwischen Zusatzobjekten und Untersuchungsobjekt oder gar innerhalb dieser Objekte, insbesondere wenigstens eines Zusatzobjekts, zu dem eine Materialinformation eine entsprechende Strukturinformation umfasst, unterschieden werden kann. Ein solches binäres oder Voxel in mehrere Materialklassen klassifizierendes Bild kann im Schritt S1 im Übrigen auch gewonnen werden, ohne dass die Oberflächen des Patienten und gegebenenfalls des wenigstens einen Zusatzobjekts vollständig erfasst werden, nachdem eine Vervollständigung der Oberfläche des Patienten anhand eines insbesondere statistisch bestimmten Patientenmodells vorgenommen werden kann, welches an die von der wenigstens einen 3D-Kamera erfassten Oberflächenanteile angepasst wird. Für Zusatzobjekte gilt, dass hier aufgrund der Sensordaten der wenigstens einen 3D-Kamera, bei denen es sich ja um optische Sensordaten handelt, identifiziert werden können, indem eine Objektinformation, die die Art des Zusatzobjekts beschreibt, ermittelt wird. Verschiedenen Objektinformationen sind dabei verschiedene dreidimensionale Computermodelle, vorliegend CAD-Modelle, (Computer-Aided/Assisted-Design-Modelle) zugeordnet. Derartige dreidimensionale Computermodelle von Zusatzobjekten können passgenau anhand der bekannten Anteile der Oberfläche des Zusatzobjekts aus den Sensordaten in ein derartiges nach Materialklassen aufteilendes Bild der Sensorinformation eingefügt werden.

In einem Schritt S2 wird anhand der Sensorinformation dann überprüft, ob Teile des Patienten und/oder wenigstens eines Zusatzobjekts aus dem nominellen Sichtfeld in die aufgrund von Projektionsbildern geplante Rekonstruktion eines Computertomographie-Bilddatensatzes störender Weise herausragen, insbesondere also in einer Ebene, in der die Aufnahmeanordnung oder zumindest der Röntgenstrahler in der Gantry eine Kreisbahn als Aufnahmetrajektorie beschreibt (bei einem zylinderförmigen nominellen Sichtfeld also aus dem Zylindermantel). Dies sei mithilfe von Fig. 2 näher erläutert.

Fig. 2 zeigt mithilfe von Paaren von Positionen 1, 2 sowie 3, 4 des Röntgenstrahlers (Positionen 1, 3) und des Röntgendetektors (Positionen 2, 4) die Bildaufnahme in unterschiedlichen Projektionsrichtungen entlang einer kreisförmigen Aufnahmetrajektorie 5, angedeutet durch die jeweiligen Strahlenfelder 6, 7. Ersichtlich definieren die Ausdehnungen der Strahlenfelder 6, 7 sowie der weiteren möglichen Strahlenfelder den nominellen Sichtbereich 8 der Computertomographieeinrichtung, das bedeutet, innerhalb des nominellen Sichtbereichs 8 befindliche Objektanteile werden vollständig durch den Röntgendetektor erfasst und können mithin, beispielsweise im Rahmen einer gefilterten Rückprojektion, korrekt rekonstruiert werden. Vorliegend ragt jedoch ein Anteil 9 des angedeuteten Untersuchungsobjekts 10 aus dem nominellen Sichtfeld 8 heraus, so dass er zwar im Strahlenfeld 7 durch den Röntgendetektor erfasst wird, jedoch nicht im Strahlenfeld 6. Wird nun, um das gesamte Untersuchungsobjekt 10 zu erfassen, einfach eine Rekonstruktion im erweiterten Sichtfeld 11 durchgeführt, das den Anteil 9 mit einschließt, könnte es zu Artefakten, insbesondere sogenannten Trunkierungsartefakten, kommen, so dass spezielle Algorithmen eingesetzt werden.

In der hier im Fall eines Herausragens aus dem nominellen Sichtfeld 8 eingesetzten Klasse von Algorithmen zur Rekonstruktion im erweiterten Sichtfeld 11 werden Projektionsbilder, die das Untersuchungsobjekt 10 trunkiert zeigen, beispielsweise gemäß dem Strahlenfeld 6, um virtuelle Projektionsdaten ergänzt, vorliegend im gestrichelt angedeuteten Bereich 12. Dabei wird, wie im Folgenden noch genauer dargelegt werden wird, eine Vorwärtsprojektion durchgeführt, die im in Fig. 2 dargestellten Fall ein möglichst genaues Wissen über die Kontur des Untersuchungsobjekts 10 erfordert, bei herausragendem Zusatzobjekt zusätzlich oder alternativ genaues Wissen über die Kontur des Zusatzobjekts.

Der Schritt S2, vgl. wiederum Fig. 1, dient nun dazu, zu entscheiden, ob solche Algorithmen zur Rekonstruktion in einem erweiterten Sichtfeld 11 benötigt werden. Dies geschieht vollständig automatisch anhand der im Schritt S1 ermittelten Sensorinformation, das bedeutet, es wird automatisch überprüft, ob sich sämtliche Anteile des Untersuchungsobjekts 10 sowie gegebenenfalls des wenigstens einen Zusatzobjekts (welches nicht röntgentransparent ist) innerhalb des nominellen Sichtfelds 8 befinden. Ist dies der Fall, wird im Schritt S3 mit einer normalen, üblichen Aufnahme und Rekonstruktion eines hier dreidimensionalen Bilddatensatzes fortgefahren, wobei die Rekonstruktion auf das nominelle Sichtfeld 8 beschränkt bleibt.

Wird jedoch festgestellt, dass ein Objekt aus dem nominellen Sichtfeld in relevanter Weise herausragt (in einer Ebene, in der die Aufnahmetrajektorie 5 verläuft), wird mit einem Schritt S4 fortgefahren, in dem eine Konturinformation ermittelt wird, die einer im Schritt S5 durchzuführenden Vorwärtsprojektion zur Ermittlung der zu ergänzenden Projektionsdaten zugrunde zu legen ist. Hierfür existieren verschiedene Ausführungsformen, die im Folgenden kurz diskutiert werden sollen und allesamt durch das Verfahren der Fig. 1 realisiert werden können.

So ist es zum einen denkbar, die Sensorinformation unmittelbar als Konturinformation zu verwenden, da sie auf äußerst verlässliche Weise mit exakt messenden optischen 3D-Kameras gewonnen wurde. Dies ist immer dann sinnvoll, wenn, beispielsweise durch Verwendung eines Patientenmodells und/oder dreidimensionaler Computermodelle, auch die nicht erfassten Anteile der Oberfläche der jeweiligen relevanten Objekte hochgenau abgeschätzt werden können.

Möglich ist es in einer alternativen Ausführungsform im Schritt S4 jedoch auch, von einer Vorabinformation, die ebenfalls die wenigstens eine relevante Kontur beschreibt, auszugehen, die durch eine initiale Rekonstruktion der ursprünglich aufgenommenen Projektionsbilder bestimmt wird. Das bedeutet, vor der Durchführung des Schrittes S4 sind in einem nicht in Fig. 1 dargestellten Schritt bereits die vorliegend zweidimensionalen Projektionsbilder des Patienten entlang der Aufnahmetrajektorie 5 aufgenommen worden, so dass sie nun, das Untersuchungsobjekt 10 und/oder gegebenenfalls ein Zusatzobjekt teilweise trunkiert zeigend, vorliegen. Nichtsdestotrotz lässt sich aus diesen Projektionsbildern auch bereits im erweiterten Sichtfeld 11 eine Rekonstruktion durchführen, die gegebenenfalls fehlerbehaftet ist, wobei, vgl. den eingangs diskutierten Stand der Technik, bereits Verfahren vorgeschlagen wurden, um die Fehleranzahl hier zumindest zu reduzieren. Diese initiale Rekonstruktion beschreibt das relevante Objekt, im Fall der Fig. 2 das Untersuchungsobjekt 10, und somit dessen Kontur auch im erweiterten Sichtfeld 11. Beispielsweise in einem schwellwertbasierten Verfahren (Schwellwertvergleich) kann aus dem in der initialen Rekonstruktion ermittelten Rekonstruktionsdatensatz beispielsweise ein binäres Bild, mithin ein in "Luft" und "Untersuchungsobjekt 10" einteilendes Materialklassen-Bild, als Vorabinformation ermittelt werden. Die hierdurch beschriebene Patientenkontur wird dann mittels eines elastischen Registrierungsalgorithmus so angepasst, dass sie nach der Registrierung mit der Lage der in der Sensorinformation vorliegenden Patientenoberfläche übereinstimmt. Dies ist insbesondere dann eine zweckmäßige Vorgehensweise, wenn durch die Sensorinformation die Kontur des Untersuchungsobjekts 10 nur teilweise hochgenau bestimmt werden konnte.

Es sei an dieser Stelle noch angemerkt, dass zur Vereinfachung der hier beschriebenen Vorgehensweisen eine Registrierung zwischen der wenigstens einen optischen 3D-Kamera und dem Koordinatensystem, in dem die Projektionsbilder mit der Aufnahmeanordnung erfasst werden, bereits vorliegen kann. Eine derartige Registrierung kann beispielsweise mittels eines in beiden Modalitäten sichtbaren Phantoms durchgeführt werden.

Im Fall eines aus dem nominellen Sichtfeld 8 herausragenden und/oder anderweitig zu berücksichtigenden Zusatzobjekts kann im Übrigen auch im Schritt S4 wiederum das bereits erwähnte dreidimensionale Computermodell herangezogen werden. Beispielsweise kann mittels der eine Objektinformation enthaltenden Sensorinformation das Zusatzobjekt identifiziert und das korrekte dreidimensionale Computermodell ausgewählt werden. Dieses dreidimensionale Computermodell, insbesondere CAD-Modell, kann nun an die initiale Rekonstruktion der Projektionsdaten (und/oder auch die Sensorinformation) bezüglich seiner Lage im Raum angepasst werden. Aus dem bezüglich seiner Lage an die Konturverläufe im Rekonstruktionsdatensatz angepassten dreidimensionalen Modell kann dann wiederum ein zumindest bezüglich des Zusatzobjekts binäres Bild (Nicht-Objekt und Objekt) erstellt werden, wobei vorteilhaft auch eine Kombination mit einem entsprechenden binären Bild des Untersuchungsobjekts 10 stattfinden kann, um ein in drei oder mehr Materialklassen eingeteiltes Materialklassen-Bild zu erhalten. Es sei angemerkt, dass das dreidimensionale Computermodell des Zusatzobjekts auch eine Materialinformation, die Schwächungseigenschaften beschreibt, aufweisen kann, welche das Zusatzobjekt gegebenenfalls weiter in Materialklassen einteilen kann, was hinsichtlich der Vorwärtsprojektion im Schritt S5 zweckmäßig ist.

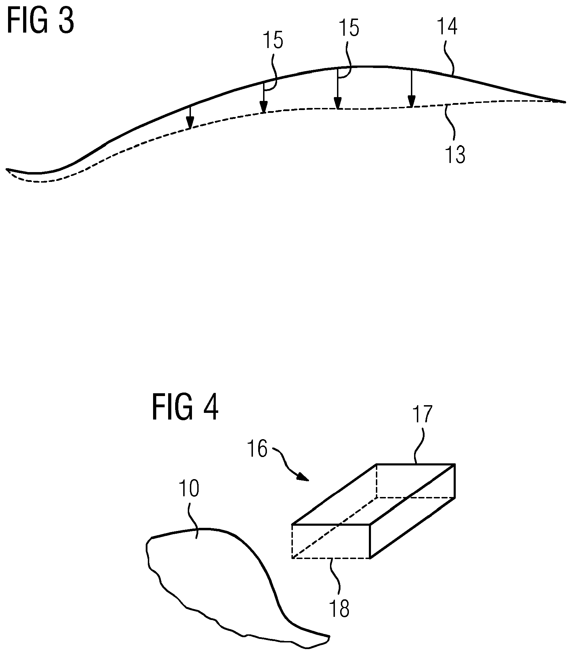

Fig. 3 zeigt Aspekte dieser verschiedenen Ansätze unter Verwendung einer Vorabinformation genauer. So ist in Fig. 3 dargestellt, dass eine in der Sensorinformation enthaltene Kontur 13 (gestrichelt) von einer in der Vorabinformation enthaltenen Kontur 14 (durchgezogen) abweichen kann und durch elastische Registrierung, vgl. Pfeile 15, entsprechend angepasst werden kann, um die Konturinformation zu erzeugen.

Fig. 4 zeigt die Ergänzung einer Kontur eines hier vereinfacht quaderförmig dargestellten Zusatzobjekts 16 durch ein dreidimensionales Computermodell. Beispielsweise können Anteile 17 (durchgezogen) der Kontur des Zusatzobjekts 16 bereits bekannt sein. Durch Einpassen des dreidimensionalen Computermodells können auch gestrichelt gezeigte, noch nicht bzw. noch nicht exakt bekannte Anteile 18 ergänzt werden.

In einem Schritt S5, vgl. wiederum Fig. 1, wird die im Schritt S4 ermittelte Konturinformation genutzt, um zur Ergänzung im Schritt S6 zu nutzende Projektionsdaten im Rahmen einer Vorwärtsprojektion zu ermitteln. Dabei können insbesondere in der vorteilhaften Ausgestaltung, in der ein Materialklassen-Bild als Konturinformation erzeugt wurde, den entsprechenden Materialklassen HU-Werte zugeordnet werden, beispielsweise dem Untersuchungsobjekt 10, also dem Patienten, der HU-Wert von Wasser und einem Zusatzobjekt 16 HU-Werte, die sich aus einer dem dreidimensionalen Computermodell zugeordneten Materialinformation ergeben. Die sich aus der Vorwärtsprojektion im Schritt S5 ergebende Ergänzung ist durch den Schritt S6 dargestellt. Hierbei können beispielsweise glatte Übergänge zu den tatsächlich gemessenen Projektionsdaten der ursprünglichen Projektionsbilder geschaffen werden.

Im Schritt S7 erfolgt dann die Rekonstruktion des Computertomographie-Bilddatensatzes, der hier aufgrund der zweidimensionalen Projektionsbilder dreidimensional ist, aus den ergänzten Projektionsbildern im erweiterten Sichtfeld 11, so dass aufgrund der genauen Ermittlung der Kontur hochqualitative Bilddaten erhalten werden.

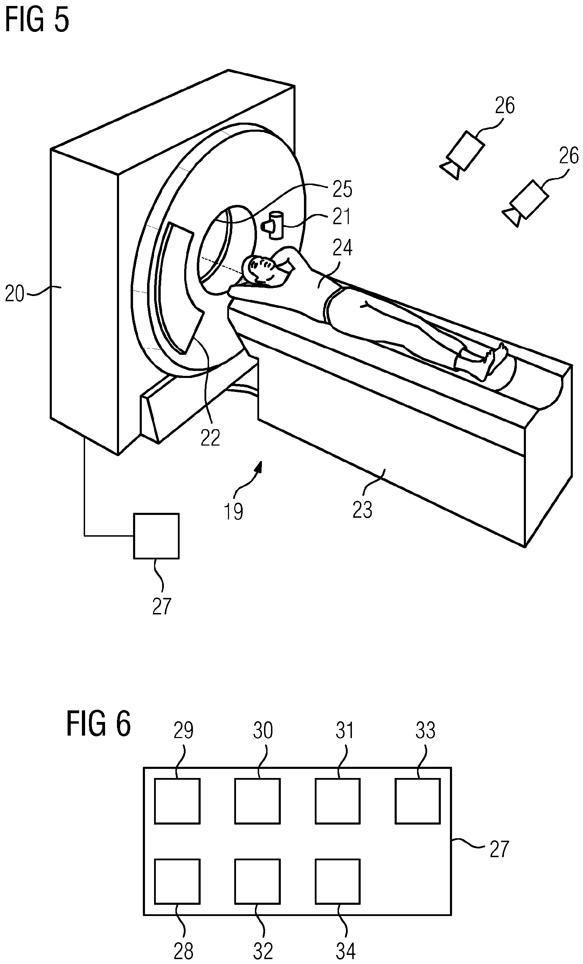

Fig. 5 zeigt eine Prinzipskizze einer erfindungsgemäßen Computertomographieeinrichtung 19. Diese umfasst, wie grundsätzlich bekannt, eine Gantry 20 in der die Aufnahmeanordnung bestehend aus einem Röntgenstrahler 21 und einem Röntgendetektor 22 drehbar gelagert ist. Der auf einem Patiententisch 23 gelagerte Patient 24 kann in eine Patientenaufnahme 25 der Gantry 20 eingefahren werden.

Die Computertomographieeinrichtung 19 umfasst nun vorliegend beispielhaft zwei deckenmontierte Kameras 26, die als optische 3D-Kameras ausgebildet sind und Sensordaten liefern, die die Oberfläche des Patienten 25 als Untersuchungsobjekt 10 sowie gegebenenfalls von Zusatzobjekten 16 beschreiben. Dabei sei darauf hingewiesen, dass die im Schritt S1 zur Sensorinformation auszuwertenden Sensordaten der Kameras 26 bei bereits für die Aufnahme der Projektionsbilder positionierten Patienten 24 aufzunehmen sind, dieser sich aber noch nicht zwangsläufig innerhalb der Patientenaufnahme 25 befinden muss, nachdem Informationen über die Stellung des motorisch betriebenen Patiententisches 23 in einer den Betrieb der Computertomographieeinrichtung 19 steuernden Steuereinrichtung 27 vorliegen.

Die Steuereinrichtung 27 ist zur Durchführung des erfindungsgemäßen Verfahrens ausgebildet, wobei deren funktionale Struktur durch Fig. 27 nochmals näher erläutert wird. Dementsprechend umfasst die Steuereinrichtung 27 neben einer grundsätzlich bekannten Aufnahmeeinheit 28, über die beispielsweise die Aufnahmeanordnung und weitere Komponenten zur Aufnahme von Projektionsbildern ansteuerbar sind, zunächst eine Sensorinformationsermittlungseinheit 29, über die die Sensorinformation gemäß Schritt S1 ermittelt werden kann. In einer Entscheidungseinheit 30 kann die Entscheidung gemäß Schritt S2 getroffen werden. Eine Konturinformationsermittlungseinheit 31 ist zur Durchführung des Schrittes S4 ausgebildet, wobei die Konturinformationsermittlungseinheit 31 hierbei bereits auf eine allgemeine Rekonstruktionseinheit 32 zur Rekonstruktion von höher dimensionalen Datensätzen aus Projektionsbildern zurückgreifen kann, in der beispielsweise unterschiedliche Rekonstruktionsalgorithmen, insbesondere auch für die Rekonstruktion im erweiterten Sichtfeld 11, bereitgestellt werden können. Die Konturinformationsermittlungseinheit 31 kann beispielsweise zur Ermittlung eines Rekonstruktionsdatensatzes, der einer Vorabinformation zugrunde zu legen ist, auf die Rekonstruktionseinheit 32 zugreifen.

Mittels einer Vorwärtsprojektionseinheit 33 kann die Vorwärtsprojektion des Schrittes S5 durchgeführt werden, während eine Ergänzungseinheit 34 zur Ergänzung der Projektionsdaten aufgrund der Vorwärtsprojektion vorgesehen ist, mithin zur Ausführung des Schrittes S6.

Die Rekonstruktionseinheit 32 dient selbstverständlich auch für die durchzuführenden Rekonstruktionen in den Schritten S3 und S7.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung, wie in den Patentansprüchen definiert, verlassen.

## Patentansprüche

1. Verfahren zur Rekonstruktion eines Bilddatensatzes aus unter unterschiedlichen Projektionsrichtungen mit einer Computertomographieeinrichtung (19) aufgenommenen Projektionsbildern eines Untersuchungsobjekts (10), wobei die Computertomographieeinrichtung (19) ein nominelles Sichtfeld (8) aufweist, das aus allen Projektionsrichtungen in den jeweiligen Projektionsbildern erfasst wird, und wobei das Untersuchungsobjekt (10) und/oder ein dem Untersuchungsobjekt (10), insbesondere dessen Lagerung, zugeordnetes Zusatzobjekt (16) sich teilweise aus dem nominellen Sichtfeld (8) heraus in ein erweitertes Sichtfeld (11) erstreckt, wobei zur Rekonstruktion des Bilddatensatzes
- eine die Kontur des Untersuchungsobjekts (10) und/oder des Zusatzobjekts (16) beschreibende Konturinformation ermittelt wird,
- die Projektionsdaten der Projektionsbilder aufgrund der Konturinformation durch Vorwärtsprojektion in Bereichen, in denen das Untersuchungsobjekt (10) und/oder das Zusatzobjekt (16) in den Projektionsdaten eines Projektionsbildes nicht erfasst wurde, ergänzt werden, und
- die Rekonstruktion aufgrund der ergänzten Projektionsdaten erfolgt,
wobei aus Sensordaten wenigstens einer das zur Aufnahme der Projektionsbilder positionierte Untersuchungsobjekt (10) und/oder das Zusatzobjekt (16) erfassenden Kamera (26) eine die Kontur des Untersuchungsobjekts (10) und/oder des Zusatzobjekts (16) wenigstens teilweise beschreibende Sensorinformation ermittelt und zur Ermittlung der Konturinformation verwendet wird,
**dadurch gekennzeichnet, dass** zunächst durch Rekonstruktion eines vorläufigen Rekonstruktionsdatensatzes aus den ursprünglichen Projektionsbildern für das erweiterte Sichtfeld (11) und Schwellwertbildung eine wenigstens die Kontur des Untersuchungsobjekts (10) und/oder des Zusatzobjekts (16) vorläufig beschreibende Vorabinformation ermittelt wird, welche aufgrund der Sensorinformation angepasst wird.

2. Verfahren nach Anspruch 1, **dadurch gekennzeichnet, dass** eine als eine 3D-Kamera (26), insbesondere eine Terahertz-Kamera (26), ausgebildete Kamera (26) verwendet wird und/oder wenigstens eine auf die Kontur des Untersuchungsobjekts (10) und/oder des Zusatzobjekts (16) bezogene 3D-Information aus Sensordaten wenigstens zweier unterschiedlich positionierter Kameras (26) und/oder aus einer Anordnungsinformation der Kamera (26) ermittelt wird und/oder außerhalb des Erfassungsbereiches einer Kamera (26) liegende Anteile der Kontur durch wenigstens eine weitere Kamera (26) erfasst werden.

3. Verfahren nach Anspruch 1 oder 2, **dadurch gekennzeichnet, dass** die Kamera (26) mit einer Aufnahmeanordnung der Computertomographieeinrichtung (19) und/oder bei mehreren Kameras (26) diese untereinander registriert sind und/oder werden.

4. Verfahren nach einem der vorangehenden Ansprüche, **dadurch gekennzeichnet, dass** ein nicht durch die wenigstens eine Kamera (26) erfassbarer, insbesondere verdeckter Anteil der Kontur des Untersuchungsobjekts (10) durch Extrapolation und/oder Interpolation, insbesondere unter Verwendung eines Patientenmodells, ermittelt wird und/oder als unbekannt markiert wird.

5. Verfahren nach einem der vorangehenden Ansprüche, **dadurch gekennzeichnet, dass** als Konturinformation eine nach Luft und Untersuchungsobjekt (10) und/oder Zusatzobjekt (16) klassifizierende binäre Einteilung wenigstens des erweiterten Sichtfelds (11) verwendet wird.

6. Verfahren nach einem der vorangehenden Ansprüche, **dadurch gekennzeichnet, dass** die Anpassung der Vorabinformation zu der Konturinformation durch elastische Registrierung der Vorabinformation auf in der Sensorinformation enthaltene Anteile der Kontur erfolgt.

7. Verfahren nach einem der vorangehenden Ansprüche, **dadurch gekennzeichnet, dass** aus den Sensordaten wenigstens eine die Art des insbesondere zur Lagerung und/oder Positionierung des Untersuchungsobjekts (10) verwendeten Zusatzobjekts (16), zu dem ein dreidimensionales Computermodell, insbesondere ein CAD-Modell, vorliegt, beschreibende Objektinformation ermittelt wird, wobei das dreidimensionale Computermodell bei der Ermittlung der die Kontur des Zusatzobjekts (16) beschreibenden Konturinformation verwendet wird.

8. Verfahren nach Anspruch 7, **dadurch gekennzeichnet, dass** aufgrund der Objektinformation das dreidimensionale Computermodell des Zusatzobjekts (16) ausgewählt wird, wonach die Kontur des Zusatzobjekts (16) gemäß der Sensorinformation und/oder der Vorabinformation zur Ermittlung der Kontur des Zusatzobjekts (16) in der Konturinformation an das dreidimensionale Computermodell des Zusatzobjekts (16), insbesondere durch elastische Registrierung, angepasst wird.

9. Verfahren nach Anspruch 7 oder 8, **dadurch gekennzeichnet, dass** das dreidimensionale Computermodell des Zusatzobjekts (16) auch eine die Schwächungseigenschaften für Röntgenstrahlung in dem Zusatzobjekt (16) beschreibende Materialinformation umfasst, welche bei der Vorwärtsprojektion berücksichtigt wird.

10. Verfahren nach einem der vorangehenden Ansprüche, **dadurch gekennzeichnet, dass** für jede Messung die Sensorinformation automatisch auf aus dem nominellen Sichtfeld (8) ragende Anteile (9) des Untersuchungsobjekts (10) und/oder des Zusatzobjekts (16) überprüft wird, wodurch automatisch die Rekonstruktion mit oder ohne erweitertes Sichtfeld (11) ausgelöst wird.

11. Computertomographieeinrichtung (19), aufweisend wenigstens eine Kamera (26) und eine zur Durchführung eines Verfahrens nach einem der vorangehenden Ansprüche ausgebildete Steuereinrichtung (27).

12. Computerprogramm, welches die Schritte eines Verfahrens nach einem der Ansprüche 1 bis 10 durchführt, wenn es auf einer Steuereinrichtung (27) einer Computertomographieeinrichtung (19) ausgeführt wird.

13. Elektronisch lesbarer Datenträger, auf dem ein Computerprogramm nach Anspruch 12 gespeichert ist.

## Claims

1. Method for the reconstruction of an image data set from projection images of an examination object (10) recorded at different projection directions with a computed tomography apparatus (19), wherein the computed tomography apparatus (19) has a nominal field of view (8) which is acquired from all projection directions in the respective projection images, and wherein the examination object (10) and/or an additional object (16) associated with the examination object (10), in particular, with its positioning, extends partially out of the nominal field of view (8) into an extended field of view (11), wherein for the reconstruction of the image data set
- an item of contour information describing the contour of the examination object (10) and/or of the additional object (16) is established,
- the projection data of the projection images is enhanced on the basis of the contour information by means of forward projection in regions in which the examination object (10) and/or the additional object (16) was not acquired in the projection data of a projection image, and
- the reconstruction takes place on the basis of the enhanced projection data,
wherein, from sensor data of at least one camera (26) acquiring the examination object (10) positioned for recording the projection images and/or the additional object (16), an item of sensor information at least partially describing the contour of the examination object (10) and/or of the additional object (16) is established and is used for establishing the contour information,
**characterised in that**, firstly through reconstruction of a provisional reconstruction data set from the original projection images for the extended field of view (11) and threshold value formation, an item of preliminary information provisionally describing at least the contour of the examination object (10) and/or of the additional object (16) is established, said preliminary information being adapted on the basis of the sensor information.

2. Method according to claim 1, **characterised in that** a camera (26) configured as a 3D camera (26), in particular, a terahertz camera (26) is used and/or at least one item of 3D information relating to the contour of the examination object (10) and/or of the additional object (16) is established from sensor data of at least two differently positioned cameras (26) and/or from an item of arrangement information of the camera (26) and/or portions of the contour lying outside the acquisition range of a camera (26) are acquired by at least one further camera (26).

3. Method according to claim 1 or 2, **characterised in that** the camera (26) is and/or will be registered with a recording arrangement of the computed tomography apparatus (19) and/or in the case of a plurality of cameras (26), they are or will be registered among one another.

4. Method according to one of the preceding claims, **characterised in that** a portion of the contour, in particular, a covered portion of the contour, of the examination object (10) not acquirable by the at least one camera (26) is established by extrapolation and/or interpolation, in particular, using a patient model and/or is marked as unknown.

5. Method according to one of the preceding claims, **characterised in that** as contour information, a binary subdivision classifying according to air and examination object (10) and/or additional object (16) at least of the extended field of view (11) is used.

6. Method according to one of the preceding claims, **characterised in that** the adaptation of the preliminary information to the contour information takes place through elastic registration of the preliminary information to components of the contour contained in the sensor information.

7. Method according to one of the preceding claims, **characterised in that** from the sensor data, at least one item of object information describing the type of the additional object (16) used, in particular, for supporting and/or for positioning the examination object (10), for which additional object a three-dimensional computer model, in particular a CAD model exists, is established, wherein the three-dimensional computer model is used in the establishment of the contour information describing the contour of the additional object (16) .

8. Method according to claim 7, **characterised in that**, on the basis of the object information, the three-dimensional computer model of the additional object (16) is selected, after which the contour of the additional object (16) is adapted, according to the sensor information and/or the preliminary information for the establishment of the contour of the additional object (16) in the contour information, to the three-dimensional computer model of the additional object (16), in particular through elastic registration.

9. Method according to claim 7 or 8, **characterised in that** the three-dimensional computer model of the additional object (16) also comprises an item of material information describing the attenuation properties for X-ray radiation in the additional object (16), said material information being taken into account in the forward projection.

10. Method according to one of the preceding claims, **characterised in that** for each scan, the sensor information is automatically checked for portions (9) of the examination object (10) and/or the additional object (16) protruding outside the nominal field of view (8), whereby the reconstruction is automatically triggered with or without an extended field of view (11).

11. Computed tomography apparatus (19), having at least one camera (26) and a control device (27) configured for carrying out a method according to one of the preceding claims.

12. Computer program which carries out the steps of a method according to one of claims 1 to 10 when said program is executed on a control device (27) of a computed tomography apparatus (19).

13. Electronically readable data carrier on which a computer program according to claim 12 is stored.

## Revendications

1. Procédé de reconstruction d'un ensemble de données d'image composé d'images de projection, enregistrées dans des directions de projection différentes par un dispositif (19) de tomodensitométrie assisté par ordinateur, d'un objet (10) à examiner, le dispositif (19) de tomodensitométrie assisté par ordinateur ayant un champ (8) de vue nominal, qui est pris à partir de toutes les directions de projection dans les images de projection respectives, et dans lequel l'objet (10) à examiner et/ou un objet (16) supplémentaire associé à l'objet (10) à examiner, notamment son logement s'étend en partie hors du champ (8) de vue nominal dans un champ (11) de vue étendu, dans lequel, pour la reconstruction du jeu de données d'image
- on détermine une information de contour décrivant le contour de l'objet (10) à examiner et/ou de l'objet (16) supplémentaire,
- on complète les données de projection des images de projection sur la base de l'information de contour par projection en avant dans des parties, dans lesquelles l'objet (10) à examiner et/ou l'objet (16) supplémentaire n'a pas été pris dans les données de projection d'une image de projection, et
- la reconstruction est effectuée sur la base des données de projection complétées,
dans lequel, à partir des données de capteur d'au moins une caméra (26), placée pour l'enregistrement des images de projection et prenant l'objet (10) à examiner et/ou l'objet (16) supplémentaire, on détermine une information de capteur décrivant, au moins en partie, le contour de l'objet (10) à examiner et/ou de l'objet (16) supplémentaire et on l'utilise pour la détermination de l'information de contour,
**caractérisé en ce que** l'on détermine d'abord, par reconstruction d'un ensemble de données de reconstruction provisoire, à partir des images de projection d'origine pour le champ (11) de vue étendu et d'une formation d'une valeur de seuil, une information préalable décrivant provisoirement au moins le contour de l'objet (10) à examiner et/ou de l'objet (16) supplémentaire, que l'on adapte sur la base de l'information de capteur.

2. Procédé suivant la revendication 1, **caractérisé en ce que** l'on utilise une caméra (26), constituée sous la forme d'une caméra (26) en 3D, notamment d'une caméra (26) térahertz et/ou **en ce que** l'on détermine au moins une information en 3D se rapportant au contour de l'objet (10) à examiner et/ou de l'objet (16) supplémentaire, à partir de données de capteur d'au moins deux caméras (26) placées différemment et/ou à partir d'une information d'agencement des caméras (26) et/ou on prend, par au moins une autre caméra (26), des parties du contour se trouvant à l'extérieur du domaine de prise d'une caméra (26).

3. Procédé suivant la revendication 1 ou 2, **caractérisé en ce que** les caméras (26) sont enregistrées par un agencement d'enregistrement du dispositif (19) de tomodensitométrie assisté par ordinateur et/ou entre elles, s'il y a plusieurs caméras (26).

4. Procédé suivant l'une des revendications précédentes, **caractérisé en ce que** l'on détermine une partie, qui ne peut pas être prise par la au moins une caméra (26), notamment recouverte, du contour de l'objet (10) à examiner, par extrapolation et/ou interpolation, en utilisant notamment un modèle de patient, et/ou on la repère comme inconnue.

5. Procédé suivant l'une des revendications précédentes, **caractérisé en ce que** l'on utilise, comme information de contour, une division binaire, classant suivant l'air et l'objet (10) à examiner et/ou l'objet (16) supplémentaire, au moins du champ (11) de vue étendu.

6. Procédé suivant l'une des revendications précédentes, **caractérisé en ce que** l'adaptation de l'information préalable à l'information de contour s'effectue par enregistrement élastique de l'information préalable à des parties du contour contenues dans l'information de capteur.

7. Procédé suivant l'une des revendications précédentes, **caractérisé en ce qu'**à partir des données de capteur, on détermine au moins une information d'objet décrivant le type de l'objet (16) supplémentaire utilisé, notamment pour le logement et/ou la mise en position de l'objet (10) à examiner, pour lequel il y a un modèle d'ordinateur à trois dimensions, notamment un modèle CAD, le modèle d'ordinateur à trois dimensions étant utilisé lors de la détermination de l'information de contour décrivant le contour de l'objet (16) supplémentaire.

8. Procédé suivant la revendication 7, **caractérisé en ce que** l'on choisit le modèle d'ordinateur en trois dimensions de l'objet (16) supplémentaire sur la base de l'information d'objet, le contour de l'objet (16) supplémentaire étant adapté, par enregistrement élastique, conformément à l'information de capteur et/ou à l'information préalable, pour la détermination du contour de l'objet (16) supplémentaire dans la formation de contour au modèle d'ordinateur à trois dimensions de l'objet (16) supplémentaire.

9. Procédé suivant la revendication 7 ou 8, **caractérisé en ce que** le modèle d'ordinateur à trois dimensions de l'objet (16) supplémentaire comprend également une information de matériau décrivant les propriétés d'affaiblissement d'un rayonnement X dans l'objet (16) supplémentaire, information qui est prise en compte dans la projection en avant.

10. Procédé suivant l'une des revendications précédentes, **caractérisé en ce que**, pour chaque mesure, on contrôle l'information de capteur automatiquement sur des parties (9), sortant du champ (8) de vue nominal, de l'objet (10) à examiner et/ou de l'objet (16) supplémentaire, grâce à quoi la reconstruction avec ou sans champ (11) de vue étendu est déclenchée automatiquement.

11. Dispositif (19) de tomodensitométrie assisté par ordinateur, comportant au moins une caméra (26) et un dispositif (27) constitué pour effectuer un procédé suivant l'une des revendications précédentes.

12. Programme d'ordinateur, qui effectue les stades d'un procédé suivant l'une des revendications 1 à 10, lorsqu'il est réalisé sur un dispositif (27) de commande d'un dispositif (19) de tomodensitométrie assisté par ordinateur.

13. Support de données déchiffrables électroniquement, sur lequel un programme d'ordinateur suivant la revendication 12 est mis en mémoire.
